# EUROPEAN PATENT APPLICATION

(11) **EP 4 796 916 A1**
(43) Date of publication of application: **26.08.2026**
(21) Application number: 26159998.9
(22) Date of filing: 23.02.2026
(51) Int. Cl.: G01N 17/02, G01N 27/07, G01N 33/18, G01R 29/12

(54) **OFFSET CORRECTION METHOD FOR POTENTIAL DIFFERENCE MEASURING APPARATUS AND POTENTIAL DIFFERENCE MEASURING APPARATUS**

(30) Priority: 25.02.2025 JP 2025028410
(71) Applicant: SHIMADZU CORPORATION, Kyoto-shi, Kyoto 604-8511 (JP); National Institute of Maritime, Port and Aviation Technology, Mitaka-shi, Tokyo 181-0004 (JP); Tokyo University of Science, Tokyo 162-8601 (JP)
(72) Inventor: MAJIMA, Yatsuse, Kyoto-shi, Kyoto 604-8511 (JP); NISHIMURA, Naoki, Kyoto-shi, Kyoto 604-8511 (JP); TOMISAKA, Masahiro, Kyoto-shi, Kyoto 604-8511 (JP); FUJIMOTO, Shuhei, Mitaka-shi, Tokyo 181-0004 (JP); YAMAJI, Toru, Mitaka-shi, Tokyo 181-0004 (JP); KOIKE, Kentaro, Mitaka-shi, Tokyo 181-0004 (JP); HASHIMOTO, Nagate, Shinjuku-ku, Tokyo 162-8601 (JP)
(74) Representative: Müller-Boré & Partner Patentanwälte PartG mbB

(57) **Abstract**

An offset correction method for a potential difference measuring apparatus (100) includes placing a pair of electrode portions (10) including a first electrode (11a) and a second electrode (12a) at a predetermined first position and measuring a first potential difference (V1), placing the pair of electrode portions (10) at a second position at which positions of the first electrode (11a) and the second electrode (12a) at the first position are reversed, and measuring a second potential difference (V2), and calculating an underwater electric potential (Vs1) in which at least an offset of a potential difference caused by a factor other than a test object (80) has been corrected, based on the first potential difference (V1) and the second potential difference (V2).

## Description

### BACKGROUND OF THE INVENTION

### Field of the Invention

The present invention relates to an offset correction method for a potential difference measuring apparatus that measures a potential difference in water, and to a potential difference measuring apparatus.

### Description of the Background Art

Conventionally, a potential difference measuring apparatus that measures a potential difference in water is known. Such a potential difference measuring apparatus is disclosed in Japanese Patent Laid-Open No. 2017-044560, for example.

Japanese Patent Laid-Open No. 2017-044560 discloses an in-liquid potential measuring system (potential difference measuring apparatus) including an in-liquid potential measuring electrode device including a measurement electrode that measures a potential in a liquid, a controller, and an amplifier. The in-liquid potential measuring system is configured such that a pair of in-liquid potential measuring electrode devices are disposed in a liquid (in the sea) to measure a potential difference in seawater. Furthermore, the in-liquid potential measuring system is configured such that the potential difference measured by the pair of in-liquid potential measuring electrode devices is amplified by the amplifier and observed by the controller.

Although not disclosed in Japanese Patent Laid-Open No. 2017-044560, the potential difference measured by the in-liquid potential measuring electrode devices (electrode portions) includes an offset (amount of deviation) of the potential difference caused by manufacturing errors, etc. Furthermore, the potential difference measured by the electrode portions is offset (deviated) due to seawater (water) temperature and dissolved oxygen concentration in seawater (water). In other words, when the potential difference in water is measured, the measured potential difference is offset (deviated) due to potential differences caused by factors other than a test object, and thus the measurement accuracy of the potential difference (underwater electric potential) caused by the test object is reduced. Therefore, a technology that enables accurate acquisition of the underwater electric potential is desired.

### SUMMARY OF THE INVENTION

The present invention is intended to solve the above problem. The present invention aims to provide an offset correction method for a potential difference measuring apparatus and a potential difference measuring apparatus capable of accurately acquiring an underwater electric potential.

In order to attain the aforementioned object, an offset correction method for a potential difference measuring apparatus according to a first aspect of the present invention is an offset correction method for a potential difference measuring apparatus including a pair of electrode portions and a potential difference measuring unit, the pair of electrode portions including a first electrode and a second electrode configured to measure a potential in water, the potential difference measuring unit being configured to measure a potential difference between the pair of electrode portions, and includes placing the pair of electrode portions at a predetermined first position and measuring a first potential difference between the pair of electrode portions, placing the pair of electrode portions at a second position at which positions of the first electrode and the second electrode at the first position are reversed, and measuring a second potential difference between the pair of electrode portions, and calculating an underwater electric potential in which at least an offset of a potential difference caused by a factor other than a test object has been corrected, based on the first potential difference and the second potential difference.

In order to attain the aforementioned object, a potential difference measuring apparatus according to a second aspect of the present invention includes an underwater electric field sensor including a pair of electrode portions and a potential difference measuring unit, the pair of electrode portions including a first electrode and a second electrode configured to measure a potential in water, the potential difference measuring unit being configured to measure a potential difference between the pair of electrode portions, and a controller configured or programmed to calculate an underwater electric potential in which at least an offset of a potential difference caused by a factor other than a test object has been corrected, based on the potential difference measured by the underwater electric field sensor. The controller is configured or programmed to place the pair of electrode portions at a predetermined first position and measure a first potential difference between the pair of electrode portions, place the pair of electrode portions at a second position at which positions of the first electrode and the second electrode at the first position are reversed and measure a second potential difference between the pair of electrode portions, and calculate the underwater electric potential in which an offset of a potential difference caused by a factor other than a test object has been corrected, based on a measured first potential difference and a measured second potential difference.

In the offset correction method for the potential difference measuring apparatus according to the first aspect and the potential difference measuring apparatus according to the second aspect, the underwater electric potential in which the offset of the potential difference caused by the factor other than the test object has been corrected is calculated based on the first potential difference measured with the pair of electrode portions placed at the first position, and the second potential difference measured with the pair of electrode portions placed at the second position at which the positions of the first electrode and the second electrode at the first position are reversed. When a potential difference is measured using the potential difference measuring apparatus, the positive and negative signs of the potential difference caused by the factor other than the test object do not change even when the positions of the pair of electrode portions are reversed. On the other hand, the positive and negative signs of the potential difference caused by the test object change when the positions of the pair of electrode portions are reversed. Therefore, regarding the first potential difference measured at the first position and the second potential difference measured at the second position, the first electrode and the second electrode are reversed such that only the potential difference caused by the test object of the measured potential differences is reversed in sign. The value of the potential difference caused by the test object and the value of the potential difference caused by the factor other than the test object change to a non-negligible extent over the long term, but change only to a substantially negligible extent over the short term. Therefore, the underwater electric potential is calculated based on the first potential difference and the second potential difference such that it is possible to correct the offset (deviation) of the potential difference caused by the factor other than the test object. Consequently, the underwater electric potential can be accurately acquired.

The foregoing and other objects, features, aspects and advantages of the present invention will become more apparent from the following detailed description of the present invention when taken in conjunction with the accompanying drawings.

### BRIEF DESCRIPTION OF THE DRAWINGS

FIG. 1 is a block diagram showing the overall configuration of a potential difference measuring apparatus.
FIG. 2 is a sectional view of a pair of electrode portions.
FIG. 3 is a schematic view for illustrating a configuration for measuring a potential difference at a first position.
FIG. 4 is a schematic view for illustrating a configuration for measuring a potential difference at a second position.
FIG. 5 is a schematic view for illustrating a configuration in which the pair of electrode portions are rotated and placed at the first position and the second position to measure a potential difference.
FIG. 6 is a schematic view for illustrating a configuration for measuring a potential difference at a predetermined angle different from that shown in FIG. 5.
FIG. 7 is a schematic view for illustrating a configuration for measuring the potential difference of a test object.
FIG. 8 is a flowchart for illustrating a process to calculate a first underwater electric potential.

### DESCRIPTION OF THE PREFERRED EMBODIMENTS

An embodiment of the present invention is hereinafter described with reference to the drawings.

### Overall Configuration of Potential Difference Measuring Apparatus

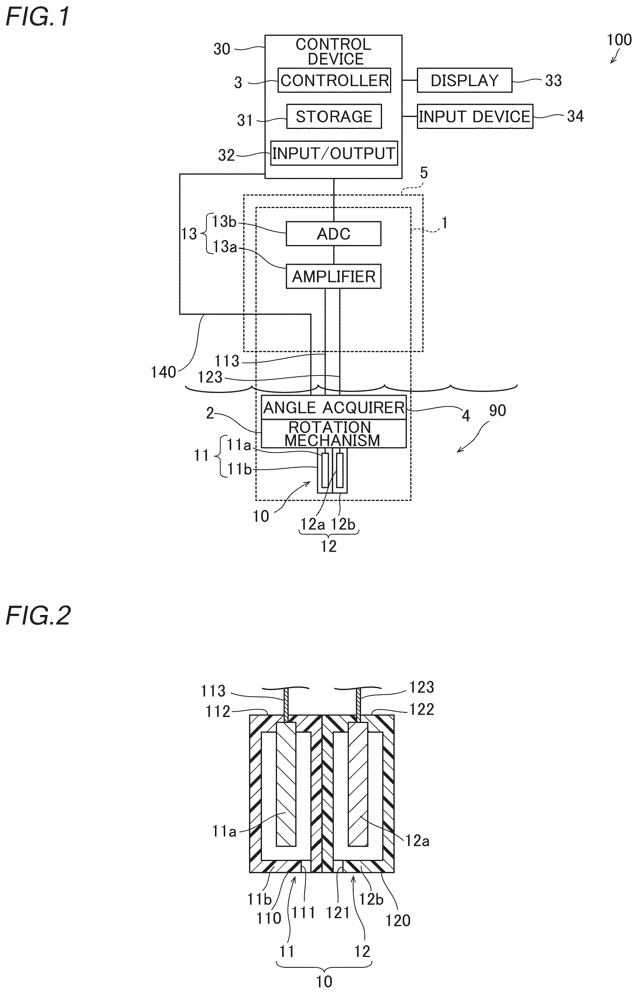

The overall configuration of a potential difference measuring apparatus 100 according to this embodiment is now described with reference to FIGS. 1 and 2.

As shown in FIG. 1, the potential difference measuring apparatus 100 includes an underwater electric field sensor 1, a rotation mechanism 2, a controller 3, and an angle acquirer 4.

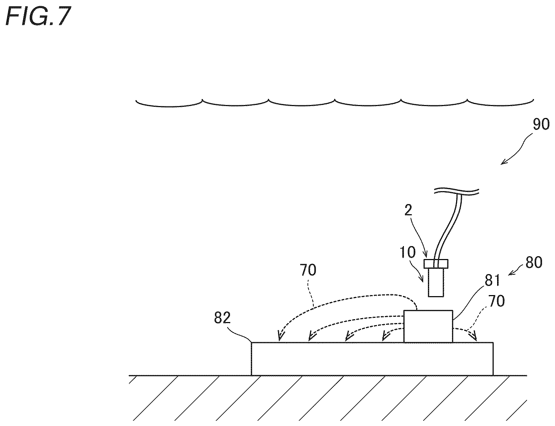

The underwater electric field sensor 1 is used to measure an underwater electric potential (UEP). The underwater electric potential (potential difference) refers to a minute potential difference in the sea 90 (in water). The underwater electric field sensor 1 includes a pair of electrode portions 10 and a potential difference measuring unit 13. The pair of electrode portions 10 include a first measuring electrode 11a and a second measuring electrode 12a that each measure a potential in water. The underwater electric field sensor 1 is configured to measure the potential difference between the pair of electrode portions 10 without contacting a test object 80 (see FIG. 7) in the sea 90. Specifically, the underwater electric field sensor 1 is configured to measure the potential difference between the first measuring electrode 11a of a first electrode portion 11 and the second measuring electrode 12a of a second electrode portion 12 without contacting the test object 80 in the sea 90. A first housing 11b of the first electrode portion 11 and a second housing 12b of the second electrode portion 12 may contact marine organisms attached to the test object 80 in the sea 90 for measurement. The "first measuring electrode 11a" and the "second measuring electrode 12a" are examples of a "first electrode" and a "second electrode" in the claims, respectively.

The potential difference measuring apparatus 100 according to this embodiment is used for at least one of position detection or corrosion measurement that targets the test object 80 "in the sea". Furthermore, the potential difference measuring apparatus 100 is not limited to detecting the position of the test object 80 "in the sea", and is not particularly limited as long as the same detects the position of the test object 80 placed "in water". The term "in water" refers to "in seawater", "in lake water", "in river water", etc., for example. Also, the term "in water" refers to "in water stored in artificial structures such as pools, tanks, and aquariums. Furthermore, "water" of the term "in water" refers to seawater, freshwater, brackish water, etc., for example, and does not include pure water.

As shown in FIG. 2, the pair of electrode portions 10 include the first electrode portion 11 and the second electrode portion 12. The pair of electrode portions 10 are used in the sea 90 (see FIG. 7) in a state in contact with seawater. The pair of electrode portions 10 are used in the vicinity of the test object 80 (see FIG. 7) in the sea 90 without contacting the test object 80. The pair of electrode portions 10 may be disposed on a self-propelled device such as an underwater robot, an underwater drone, or an autonomous unmanned underwater vehicle, for example, or may be held by a diver.

The first electrode portion 11 includes the first measuring electrode 11a and the first housing 11b. The first measuring electrode 11a is configured to measure a potential in the sea 90. As an example, the first measuring electrode 11a includes a silver-silver chloride electrode containing silver (Ag) and silver chloride (AgCl). The first measuring electrode 11a is connected to a first cable 113. The first measuring electrode 11a is connected to the potential difference measuring unit 13 (see FIG. 1) via the first cable 113.

The first measuring electrode 11a is disposed inside the first housing 11b. The first measuring electrode 11a has a cylindrical shape and extends in the longitudinal direction of the first housing 11b. The first measuring electrode 11a is aligned with the second measuring electrode 12a at a predetermined interval in the sea 90. The first measuring electrode 11a and the second measuring electrode 12a are disposed to maintain a constant interval therebetween.

The first housing 11b is configured to cover the first measuring electrode 11a. The first housing 11b is made of an insulating material such as resin. The first housing 11b has a cylindrical shape, for example. Specifically, the first housing 11b has a rectangular cylindrical shape. A first opening 111 communicating with the outside is formed in a first end face 110 of the first housing 11b on the first side. The first opening 111 is configured to enable external seawater to flow into the first housing 11b. When the potential difference measuring apparatus 100 is in use, the first measuring electrode 11a is immersed in seawater that flows into the first housing 11b through the first opening 111. The first cable 113 is inserted into a third end face 112 of the first housing 11b on the second side opposite to the first end face 110.

The second electrode portion 12 includes the second measuring electrode 12a and the second housing 12b. The second measuring electrode 12a is disposed inside the second housing 12b. The second measuring electrode 12a is connected to the potential difference measuring unit 13 (see FIG. 1) via a second cable 123. The second housing 12b also has a rectangular cylindrical shape, for example. A second opening 121 communicating with the outside is formed in a second end face 120 of the second housing 12b on the first side. The second cable 123 is inserted into a fourth end face 122 of the second housing 12b on the second side opposite to the second end face 120. The remaining configurations of the second electrode portion 12 are similar to those of the first electrode portion 11, and thus detailed description thereof is omitted.

The second electrode portion 12 is disposed adjacent to the first electrode portion 11. Specifically, the pair of electrode portions 10 are integrally provided such that the side surface of the first housing 11b of the first electrode portion 11 closer to the second electrode portion 12 contacts the side surface of the second housing 12b of the second electrode portion 12 closer to the first electrode portion 11. The pair of electrode portions 10 are integrally formed such that the first opening 111 of the first electrode portion 11 and the second opening 121 of the second electrode portion 12 are spaced a predetermined distance apart.

As shown in FIG. 1, the potential difference measuring unit 13 is configured to measure the potential difference between the pair of electrode portions 10. Specifically, the potential difference measuring unit 13 is configured to measure the potential difference (underwater electric potential) between the first measuring electrode 11a of the first electrode portion 11 and the second measuring electrode 12a of the second electrode portion 12 without contacting the test object 80 (see FIG. 7) placed in water. In other words, the potential difference measuring unit 13 is configured to measure the potential difference between the first measuring electrode 11a and the second measuring electrode 12a in a non-contact state with the test object 80.

The potential difference measuring unit 13 is housed inside a main housing 5. The main housing 5 is placed on land, on the sea, in the sea 90, inside a marine vessel, or on a self-propelled device such as an underwater robot, an underwater drone, or an autonomous unmanned underwater vehicle, for example. When the main housing 5 is placed on the sea or in the sea 90 and contacts seawater, the inside of the main housing 5 is sealed to prevent water from entering. The potential difference measuring unit 13 may be placed inside a housing other than the main housing 5.

The potential difference measuring unit 13 includes an amplifier 13a and an AD converter (ADC) 13b. The amplifier 13a is configured to generate a signal obtained by amplifying the potential difference between the first measuring electrode 11a and the second measuring electrode 12a. One of a pair of input terminals of the amplifier 13a is connected to the first measuring electrode 11a via the first cable 113. The other of the pair of input terminals of the amplifier 13a is connected to the second measuring electrode 12a via the second cable 123.

The AD converter 13b is configured to convert the signal amplified by the amplifier 13a into a digital signal and output the converted digital signal to the controller 3. Thus, the potential difference measuring unit 13 outputs the potential difference between the first measuring electrode 11a and the second measuring electrode 12a to the controller 3. The AD converter 13b is connected to the amplifier 13a and the controller 3.

The rotation mechanism 2 is configured to rotate the pair of electrode portions 10. The rotation mechanism 2 includes a holder that holds the pair of electrode portions 10, a driving source that generates a driving force to rotate the holder, and a driving force transmission member that transmits the driving force from the driving source to the holder.

The controller 3 is configured or programmed to calculate a first underwater electric potential Vs1 in which at least offsets (deviations) of potential differences caused by factors other than the test object 80 have been corrected, based on the potential difference measured by the underwater electric field sensor 1. The controller 3 is also configured to calculate a second underwater electric potential Vs2 in a direction different from the direction of the first underwater electric potential Vs1. The controller 3 includes a processor such as a CPU (Central Processing Unit) that performs computational processing, and a memory that temporarily stores data during computations. The first underwater electric potential Vs1 is an example of an "underwater electric potential" in the claims.

The controller 3 is provided in a control device 30. The control device 30 includes a PC (personal computer), for example. The control device 30 includes the controller 3, a storage 31, and an input/output 32. The control device 30 is connected to a display 33 and an input device 34.

The storage 31 includes a volatile storage and a nonvolatile storage. The input/output 32 includes various interfaces for inputting and outputting signals to and from the control device 30. The input/output 32 is connected to the display 33 and the input device 34. The display 33 is a liquid crystal display, for example. The input device 34 includes a keyboard, a mouse, etc. The controller 3 acquires the first underwater electric potential Vs1 and the second underwater electric potential Vs2 via the input/output 32.

The control device 30 is placed on land, on the sea, inside a marine vessel, etc. The controller 3 may not be provided in the control device 30. The controller 3 may be housed inside the main housing 5, or may be placed inside a housing other than the main housing 5, for example.

The angle acquirer 4 is configured to acquire the rotation angle when the pair of electrode portions 10 are rotated by the rotation mechanism 2. Specifically, the angle acquirer 4 acquires the angle of the pair of electrode portions 10 rotated by the rotation mechanism 2 at a predetermined sampling rate, with a first position (described below) as a reference. The angle acquirer 4 is also connected to the controller 3 via a cable 140. The angle acquirer 4 transmits the acquired angle to the controller 3. The angle acquirer 4 includes a rotary encoder, for example.

### Calculation of Underwater Electric Potential by Controller

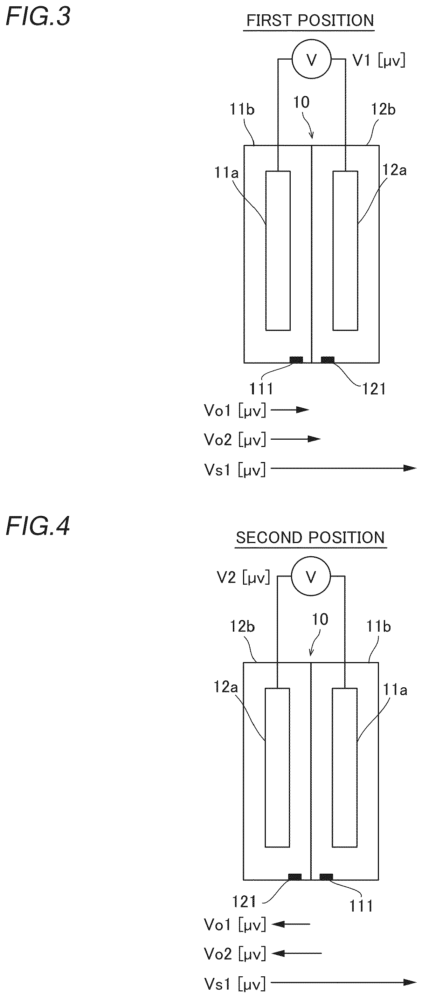

A configuration in which the controller 3 (see FIG. 1) calculates the first underwater electric potential Vs1 and the second underwater electric potential Vs2 is now described with reference to FIGS. 3 to 6.

FIG. 3 shows a state in which the pair of electrode portions 10 are placed at the first position. In this embodiment, the controller 3 places the pair of electrode portions 10 at a predetermined first position and measures a first potential difference V1 between the pair of electrode portions 10. Specifically, the controller 3 acquires a first potential difference V1 [µV] between the first measuring electrode 11a and the second measuring electrode 12a output from the underwater electric field sensor 1 placed at the first position.

The first potential difference V1 includes the first underwater electric potential Vs1, which is the underwater electric potential of the test object 80 (see FIG. 7), and offsets (amounts of deviation) of potential differences caused by factors other than the test object 80. Specifically, the first potential difference V1 includes, as offsets of potential differences caused by factors other than the test object 80, an offset of a potential difference Vo1 caused by individual differences between the first measuring electrode 11a and the second measuring electrode 12a, and an offset of a potential difference Vo2 caused by the surrounding environment of the pair of electrode portions 10. The first potential difference V1 can be expressed as the following equation (1): $V 1 = Vs 1 + Vo 1 + Vo 2$ where V1 represents a first potential difference, Vs1 represents the first underwater electric potential, Vo1 represents the potential difference caused by individual differences between the first measuring electrode 11a and the second measuring electrode 12a, and Vo2 represents the potential difference caused by the surrounding environment of the pair of electrode portions 10.

Vo1 refers to the potential difference caused by individual differences between the first measuring electrode 11a and the second measuring electrode 12a, and thus it is difficult to measure Vo1 directly. Furthermore, Vo1 changes over time as the underwater electric field sensor 1 is used. Meanwhile, Vo2 refers to the potential difference caused by the surrounding environment of the pair of electrode portions 10. Vo2 changes depending on, for example, the water temperature or dissolved oxygen concentration in the surrounding environment of the pair of electrode portions 10. Therefore, Vo2 changes over time, for example. For example, Vo2 can change by several hundred microvolts over the course of about 10 minutes. Therefore, when the first potential difference V1 is measured using the first measuring electrode 11a and the second measuring electrode 12a, it is difficult to accurately acquire the first underwater electric potential Vs1.

Therefore, in this embodiment, as shown in FIG. 4, the controller 3 uses the rotation mechanism 2 to place the pair of electrode portions 10 at a second position at which the positions of the first measuring electrode 11a and the second measuring electrode 12a at the first position are reversed, and measure a second potential difference V2 between the pair of electrode portions 10. Specifically, after the first potential difference V1 is measured, the controller 3 rotates the pair of electrode portions 10 by 180 degrees using the rotation mechanism 2 to place the pair of electrode portions 10 at the second position, and measures the second potential difference V2.

The second potential difference V2 measured when the pair of electrode portions 10 are placed at the second position also includes the first underwater electric potential Vs1, an offset of the potential difference Vo1 caused by individual differences between the first measuring electrode 11a and the second measuring electrode 12a, and an offset of the potential difference Vo2 caused by the surrounding environment of the pair of electrode portions 10. When a potential difference is measured using the potential difference measuring apparatus 100, the positive and negative signs of the potential differences caused by factors other than the test object 80 do not change even when the positions of the pair of electrode portions 10 are reversed. On the other hand, regarding the potential difference caused by the test object 80, the positions of the first measuring electrode 11a and the second measuring electrode 12a at the second position are reversed relative to their positions at the first position, and thus the positive and negative signs of the first underwater electric potential Vs1 are reversed. In other words, regarding the second potential difference V2, the value of the underwater electric potential is negative. The second potential difference V2 can be expressed as the following equation (2): $V 2 = - Vs 1 + Vo 1 + Vo 2$ where V2 represents the second potential difference.

As shown in the above equations (1) and (2), regarding the first potential difference V1 and the second potential difference V2, only the first underwater electric potential Vs1 is reversed in sign, and thus the first underwater electric potential Vs1 can be calculated based on the first potential difference V1 and the second potential difference V2. The first underwater electric potential Vs1, the potential difference Vo1 caused by individual differences between the first measuring electrode 11a and the second measuring electrode 12a, and the potential difference Vo2 caused by the surrounding environment of the pair of electrode portions 10 change to a non-negligible extent over the long term, but change only to a substantially negligible extent over the short term. Therefore, the first potential difference V1 and the second potential difference V2 are measured at time intervals within which such changes are substantially negligible. For example, the controller 3 measures the second potential difference V2 within 10 minutes after measuring the first potential difference V1.

Then, based on the measured first potential difference V1 and second potential difference V2, the controller 3 calculates the first underwater electric potential Vs1 in which offsets of potential differences caused by factors other than the test object 80 have been corrected. Specifically, the controller 3 calculates a difference between the value of the first potential difference V1 and the value of the second potential difference V2 to calculate the first underwater electric potential Vs1 in which offsets of potential differences caused by factors other than the test object 80 have been corrected. More specifically, the controller 3 calculates the difference between the value of the first potential difference V1 and the value of the second potential difference V2, and then divides the difference by two to calculate the first underwater electric potential Vs1. In other words, the controller 3 calculates the first underwater electric potential Vs1 based on the following equation (3): $Vs 1 = \left(V1-V2\right) / 2$

The controller 3 measures the second potential difference V2 at the same location at which the first potential difference V1 was measured, with the arrangement of the pair of electrode portions 10 changed from the first position to the second position. The same location at which the first potential difference V1 was measured includes not only a location at which the first measuring electrode 11a and the second measuring electrode 12a are completely reversed between the first and second positions, but also a location allowing some positional deviation.

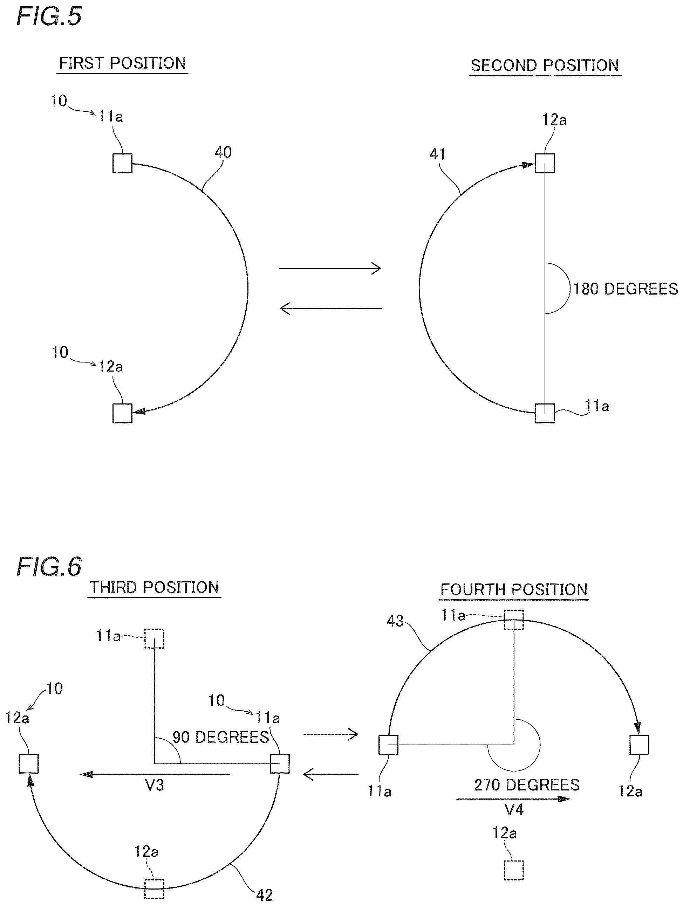

In this embodiment, as shown in FIG. 5, the controller 3 (see FIG. 1) measures the first potential difference V1 and the second potential difference V2 while rotating the pair of electrode portions 10 (the first measuring electrode 11a and the second measuring electrode 12a) by 180 degrees to switch between the first position and the second position. That is, the controller 3 controls the rotation mechanism 2 (see FIG. 1) while the pair of electrode portions 10 are at the first position to rotate the pair of electrode portions 10 by 180 degrees along an arrow 40 to place the pair of electrode portions 10 at the second position.

Furthermore, the controller 3 controls the rotation mechanism 2 while the pair of electrode portions 10 are at the second position to rotate the pair of electrode portions 10 by 180 degrees along an arrow 41 to place the pair of electrode portions 10 at the first position.

In this embodiment, the controller 3 acquires the first potential difference V1 and the second potential difference V2 while rotating the pair of electrode portions 10 using the rotation mechanism 2. In such a case, the controller 3 controls the rotation mechanism 2 to rotate the pair of electrode portions 10 at a speed that enables the first potential difference V1 and the second potential difference V2 to be acquired when the pair of electrode portions 10 are located at the first and second positions. When acquiring the potential difference while rotating the pair of electrode portions 10 using the rotation mechanism 2, the controller 3 acquires the potential difference measured by the underwater electric field sensor 1 as the first potential difference V1 each time the rotation angle acquired by the angle acquirer 4 (see FIG. 1) reaches 0 degrees. Furthermore, when acquiring the potential difference while rotating the pair of electrode portions 10 using the rotation mechanism 2, the controller 3 acquires the potential difference measured by the underwater electric field sensor 1 as the second potential difference V2 each time the rotation angle acquired by the angle acquirer 4 reaches 180 degrees from the first position. The controller 3 controls the rotation mechanism 2 to rotate the pair of electrode portions 10 at a speed that enables the pair of electrode portions 10 to acquire the potential difference at the positions of 0 degrees and 180 degrees.

The controller 3 is configured to calculate the first underwater electric potential Vs1 each time the first potential difference V1 and the second potential difference V2 are acquired. That is, based on the first potential difference V1 measured at each of a plurality of predetermined angles, which are different from each other, and the second potential difference V2 measured by reversing the positions of the pair of electrode portions 10 at each of the plurality of predetermined angles, the controller 3 calculates the first underwater electric potential Vs1 at each of the plurality of predetermined angles, in which offsets of potential differences caused by factors other than the test object 80 have been corrected.

In this embodiment, the controller 3 stores the calculated first underwater electric potential Vs1 in the storage 31 (see FIG. 1). The controller 3 may associate the calculated first underwater electric potential Vs1 with the position of the test object 80 (see FIG. 7) corresponding to the first position and store it in the storage 31. The controller 3 may also calculate the first underwater electric potential Vs1 in real time when the underwater electric field sensor 1 outputs a potential difference, and display the calculated first underwater electric potential Vs1 in real time on the display 33 (see FIG. 1).

As shown in FIG. 6, it is also possible to measure potential differences (a third potential difference V3 and a fourth potential difference V4) at positions rotated 90 degrees clockwise from the first and second positions shown in FIG. 5, and calculate the second underwater electric potential Vs2 from a difference between these potential differences. That is, in this embodiment, the underwater electric potential can be calculated for each of the plurality of predetermined angles by rotating the pair of electrode portions 10 and calculating a difference between the electric field measured at any of the plurality of predetermined angles and the electric field measured at a position rotated 180 degrees from each of the plurality of predetermined angles (i.e., by reversing the positions of the pair of electrode portions 10). Consequently, a secondary effect of being able to measure the underwater electric potential at each of a plurality of predetermined angles can be obtained by simply providing the pair of electrode portions 10.

### Measurement of Underwater Electric Potential of Test Object Using Potential Difference Measuring Apparatus

Measurement of the first underwater electric potential Vs1 of the test object 80 using the potential difference measuring apparatus 100 is now described with reference to FIG. 7.

As shown in FIG. 7, an undersea structure 82 is placed on the seabed. The undersea structure 82 is made of a steel material with iron (Fe) as its main component, for example. A sacrificial anode 81 is installed in contact with the undersea structure 82. The sacrificial anode 81 is made of zinc (Zn), for example.

In this embodiment, the potential difference measuring apparatus 100 measures the potential difference in water while being disposed on a self-propelled undersea device such as an underwater robot, an underwater drone, or an autonomous unmanned underwater vehicle, for example, or being held by a diver.

The sacrificial anode 81 made of zinc (Zn) has a higher ionization tendency than the undersea structure 82 made of a steel material, with iron (Fe) as its main component. In other words, the sacrificial anode 81 made of zinc (Zn) is more likely to ionize than the undersea structure 82 made of a steel material. Therefore, the sacrificial anode 81 oxidizes (dissolves and corrodes) while supplying a corrosion protection current 70 to the undersea structure 82. Consequently, corrosion of the undersea structure 82 is reduced or prevented.

In this embodiment, the controller 3 measures the potential differences (the first potential difference V1 to the fourth potential difference V4) based on the corrosion protection current 70 flowing from the sacrificial anode 81 to the undersea structure 82. Then, the controller 3 calculates the first underwater electric potential Vs1 and the second underwater electric potential Vs2.

Furthermore, the controller 3 may detect the position of the test object 80 using the calculated first underwater electric potential Vs1 and second underwater electric potential Vs2.

### Offset Correction Process of Potential Difference Measuring Apparatus

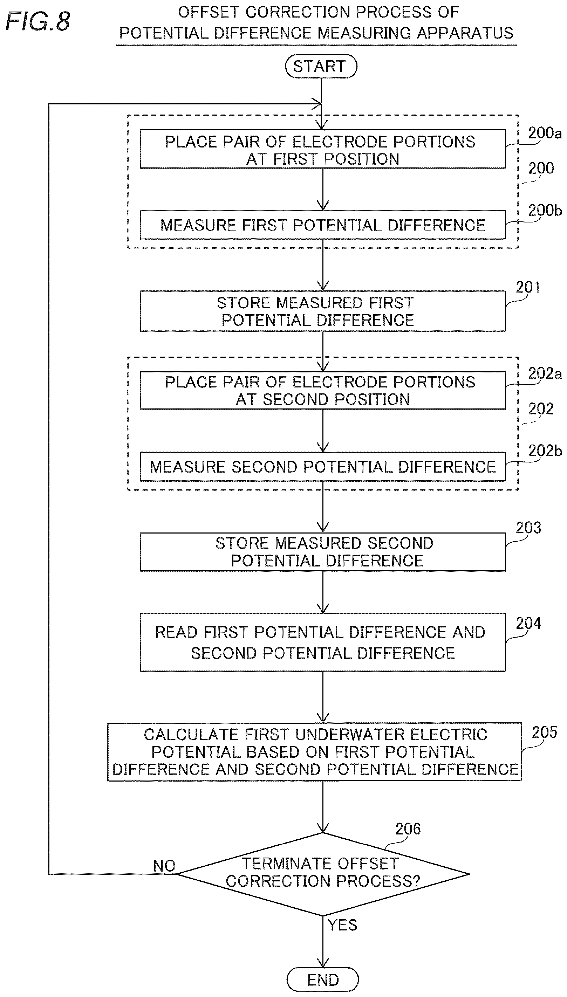

Next, a process by which the potential difference measuring apparatus 100 (controller 3) corrects an offset of the first underwater electric potential Vs1 is described with reference to FIG. 8.

In step 200, the controller 3 places the pair of electrode portions 10 at the predetermined first position and measures the first potential difference V1 between the pair of electrode portions. Specifically, in step 200a, the controller 3 controls the driving source of the rotation mechanism 2 to place the pair of electrode portions 10 at the first position. Then, in step 200b, the controller 3 measures the first potential difference V1 at the first position.

Next, in step 201, the controller 3 stores the measured first potential difference V1 in the storage 31.

Next, in step 202, the controller 3 places the pair of electrode portions 10 at the second position at which the positions of the first measuring electrode 11a and the second measuring electrode 12a at the first position are reversed, and measures the second potential difference V2 between the pair of electrode portions. Specifically, in step 202a, the controller 3 controls the driving source of the rotation mechanism 2 to rotate the pair of electrode portions 10 by 180 degrees to place the pair of electrode portions 10 at the second position. Then, in step 202b, the controller 3 measures the second potential difference V2 at the second position. In this embodiment, the controller 3 rotates the pair of electrode portions 10 by 180 degrees from a state in which the pair of electrode portions 10 are placed at the first position to place the pair of electrode portions 10 at the second position, and measures the second potential difference V2. Furthermore, in this embodiment, the controller 3 measures the second potential difference V2 at the same location at which the first potential difference V1 was measured, with the arrangement of the pair of electrode portions 10 changed from the first position to the second position.

Next, in step 203, the measured second potential difference V2 is stored in the storage 31.

Next, in step 204, the controller 3 reads the first potential difference V1 and the second potential difference V2 stored in the storage 31 from the storage 31.

Next, in step 205, the controller 3 calculates the first underwater electric potential Vs1 in which at least offsets of potential differences caused by factors other than the test object 80 have been corrected, based on the first potential difference V1 and the second potential difference V2. In this embodiment, the controller 3 calculates the difference between the value of the first potential difference V1 and the value of the second potential difference V2 to calculate the first underwater electric potential Vs1 in which offsets of potential differences caused by factors other than the test object 80 have been corrected. Specifically, the controller 3 calculates the difference between the value of the first potential difference V1 and the value of the second potential difference V2 to calculate the first underwater electric potential Vs1 in which an offset of the potential difference Vo1 caused by individual differences between the first measuring electrode 11a and the second measuring electrode 12a and an offset of the potential difference Vo2 caused by the surrounding environment of the pair of electrode portions 10 have been corrected. The controller 3 calculates the difference between the value of the first potential difference V1 and the value of the second potential difference V2, and then divides the calculated difference by two to calculate the first underwater electric potential Vs1.

Next, in step 206, the controller 3 determines whether or not to terminate the offset correction process. For example, the controller 3 determines whether or not to terminate the offset correction process based on whether or not an operation to terminate the offset correction process has been input. When an operation to terminate the offset correction process has been input, the process is terminated. When an operation to terminate the offset correction process has not been input, the process advances to step 200.

That is, step 200 of measuring the first potential difference V1 is performed each time the angle between the pair of electrode portions 10 reaches a predetermined angle (e.g., 0 degrees) when the potential difference is acquired while the pair of electrode portions 10 are rotated. Furthermore, step 202 of measuring the second potential difference V2 is performed each time the angle between the pair of electrode portions 10 reaches a predetermined angle + 180 degrees when the potential difference is acquired while the pair of electrode portions 10 are rotated. Therefore, step 205 of calculating the first underwater electric potential Vs1 is performed each time the first potential difference V1 and the second potential difference V2 are acquired. In other words, in step 205 of calculating the first underwater electric potential Vs1, the controller 3 calculates the first underwater electric potential Vs1 at each of the plurality of predetermined angles that are different from each other, in which offsets of potential differences caused by factors other than the test object 80 have been corrected, based on the first potential difference V1 measured at each of the plurality of predetermined angles and the second potential difference V2 measured by reversing the positions of the pair of electrode portions 10 at each of the plurality of predetermined angles.

### Modified Examples

The embodiment disclosed this time must be considered as illustrative in all points and not restrictive. The scope of the present invention is not shown by the above description of the embodiment but by the scope of claims for patent, and all modifications (modified examples) within the meaning and scope equivalent to the scope of claims for patent are further included.

For example, the controller may calculate the first underwater electric potential in which offsets of potential differences caused by factors other than the test object have been corrected, without calculating the difference between the first potential difference and the second potential difference. In such a case, the first underwater electric potential in which offsets of the potential differences caused by factors other than the test object have been corrected may be calculated by calculating the potential differences caused by factors other than the test object using the following equation (9) and then subtracting the potential differences caused by factors other than the test object from the first potential difference using the following equation (10). $Vo 1 + Vo 2 = \left(V1+V2\right) / 2$ $Vs 1 = V 1 - \left(Vo1+Vo2\right)$

Furthermore, for example, the controller may calculate the second underwater electric potential in which offsets of the potential differences caused by factors other than the test object have been corrected using the following equations (11) and (12). $Vo 1 + Vo 2 = \left(V3+V4\right) / 2$ $Vs 2 = V 3 - \left(Vo1+Vo2\right)$

Furthermore, for example, the controller may display, on the display, the value that is not divided by two as the first underwater electric potential after calculating the difference between the value of the first potential difference and the value of the second potential difference. When the difference between the value of the first potential difference and the value of the second potential difference is displayed without being divided by two, it is preferable to also display that this is the case.

Furthermore, for example, the potential difference measuring apparatus may not include the rotation mechanism. In such a case, for example, the pair of electrode portions may be provided on a moving body such as an underwater drone, and the controller may move the moving body to reverse the arrangement of the pair of electrode portions at the same location at which the first potential difference was measured, and measure the second potential difference.

Furthermore, for example, the pair of electrode portions may not be integrally formed.

Furthermore, for example, the pair of electrode portions may be configured such that an opening-to-opening distance between the respective openings provided in the pair of electrode portions is variable.

Furthermore, for example, the controller may switch the positions of the pair of electrode portions from the first position to the second position by rotating the pair of electrode portions 180 degrees clockwise, and then switch the positions of the pair of electrode portions from the second position to the first position by rotating the pair of electrode portions 180 degrees counterclockwise. As long as the pair of electrode portions can be switched between the first position and the second position, the pair of electrode portions may be moved (rotated) in any manner. The same applies to the third and fourth positions.

Furthermore, for example, the controller may not measure the first potential difference and the second potential difference while rotating the pair of electrode portions. In such a case, the controller may calculate the first underwater electric potential by measuring the first potential difference once at the first position and measuring the second potential difference once at the second position. The same applies to measurement of the third potential difference and the fourth potential difference.

Furthermore, for example, the rotation mechanism may be configured to enable a user to rotate the pair of electrode portions. In such a case, the rotation mechanism preferably includes a mechanism configured to fix the pair of electrode portions at each of the first position and the second position.

Furthermore, for example, the controller only needs to calculate at least the first underwater electric potential, and does not need to calculate the second underwater electric potential.

Furthermore, for example, the pair of electrode portions may include a plurality of pairs of electrode portions. That is, the pairs of electrode portions may include a first measuring electrode, a second measuring electrode, and a third measuring electrode, the first measuring electrode may be shared, a first pair of electrode portions may measure a potential difference between the first measuring electrode and the second measuring electrode, and a second pair of electrode portions may measure a potential difference between the first measuring electrode and the third measuring electrode. In such a case, the third measuring electrode may be disposed in a direction perpendicular to a direction in which the second measuring electrode is located relative to the first measuring electrode.

While the offset correction process operations performed by the controller are described using a flowchart in a flow-driven manner in which processes are performed in order along a process flow, the present invention is not limited to this. In the present invention, the offset correction process operations performed by the controller may alternatively be performed in an event-driven manner in which the processes are performed on an event basis. In this case, the offset correction process operations performed by the controller may be performed in a complete event-driven manner or in a combination of an event-driven manner and a flow-driven manner.

### Aspects

It will be appreciated by those skilled in the art that the exemplary embodiments described above are specific examples of the following aspects.

### (Item 1)

An offset correction method for a potential difference measuring apparatus, the potential difference measuring apparatus including a pair of electrode portions and a potential difference measuring unit, the pair of electrode portions including a first electrode and a second electrode configured to measure a potential in water, the potential difference measuring unit being configured to measure a potential difference between the pair of electrode portions, the offset correction method comprising:
placing the pair of electrode portions at a predetermined first position and measuring a first potential difference between the pair of electrode portions;
placing the pair of electrode portions at a second position at which positions of the first electrode and the second electrode at the first position are reversed, and measuring a second potential difference between the pair of electrode portions; and
calculating an underwater electric potential in which at least an offset of a potential difference caused by a factor other than a test object has been corrected, based on the first potential difference and the second potential difference.

When a potential difference is measured using the potential difference measuring apparatus, the positive and negative signs of the potential difference caused by the factor other than the test object do not change even when the positions of the pair of electrode portions are reversed. On the other hand, the positive and negative signs of the potential difference caused by the test object change when the positions of the pair of electrode portions are reversed. Therefore, regarding the first potential difference measured at the first position and the second potential difference measured at the second position, the first electrode (first measuring electrode) and the second electrode (second measuring electrode) are reversed such that only the potential difference caused by the test object of the measured potential differences is reversed in sign. The value of the potential difference caused by the test object and the value of the potential difference caused by the factor other than the test object change to a non-negligible extent over the long term, but change only to a substantially negligible extent over the short term. Therefore, the underwater electric potential (first underwater electric potential) is calculated based on the first potential difference and the second potential difference such that it is possible to correct the offset (deviation) of the potential difference caused by the factor other than the test object. Consequently, the underwater electric potential can be accurately acquired.

### (Item 2)

The offset correction method for the potential difference measuring apparatus according to item 1, wherein the calculating of the underwater electric potential includes calculating the underwater electric potential in which the offset of the potential difference caused by the factor other than the test object has been corrected by calculating a difference between a value of the first potential difference and a value of the second potential difference.

The difference between the value of the first potential difference and the value of the second potential difference is calculated such that the underwater electric potential (first underwater electric potential) in which the offset of the potential difference caused by the factor other than the test object has been corrected can be easily acquired. Consequently, the underwater electric potential can be easily and accurately acquired.

### (Item 3)

The offset correction method for the potential difference measuring apparatus according to item 2, wherein the calculating of the underwater electric potential includes calculating the underwater electric potential in which an offset of a potential difference caused by individual differences between the first electrode and the second electrode, and an offset of a potential difference caused by a surrounding environment of the pair of electrode portions have been corrected by calculating the difference between the value of the first potential difference and the value of the second potential difference.

The potential difference measured by the pair of electrode portions includes an offset of a potential difference caused by individual differences between the first electrode (first measuring electrode) and the second electrode (second measuring electrode) during manufacturing. The potential difference measured by the pair of electrode portions also includes an offset of a potential difference caused by a factor such as the water temperature or dissolved oxygen concentration in the surrounding environment of the pair of electrode portions. Therefore, with the above configuration, it is possible to easily calculate the underwater electric potential (first underwater electric potential) in which the offset of the potential difference caused by individual differences between the first electrode (first measuring electrode) and the second electrode (second measuring electrode) and the offset of the potential difference caused by the surrounding environment of the pair of electrode portions have been corrected. Consequently, even when it is difficult to individually measure the potential difference caused by individual differences between the first electrode (first measuring electrode) and the second electrode (second measuring electrode) and the potential difference caused by the surrounding environment of the pair of electrode portions, it is possible to easily acquire the underwater electric potential in which the offset of the potential difference caused by individual differences between the first electrode (first measuring electrode) and the second electrode (second measuring electrode) and the offset of the potential difference caused by the surrounding environment of the pair of electrode portions have been corrected.

### (Item 4)

The offset correction method for the potential difference measuring apparatus according to any one of items 1 to 3, wherein the calculating of the underwater electric potential includes calculating the underwater electric potential by calculating the difference between the value of the first potential difference and the value of the second potential difference and then dividing the difference by two.

The positive and negative signs of the underwater electric potential are reversed for the first and second potential differences, and the positive and negative signs of the potential difference caused by individual differences between the first electrode (first measuring electrode) and the second electrode (second measuring electrode) are equal to the positive and negative signs of the potential difference caused by the surrounding environment of the pair of electrode portions. Therefore, when the difference between the first potential difference value and the second potential difference value is calculated, a value twice the underwater electric potential is calculated. Therefore, as described above, the difference between the value of the first potential difference and the value of the second potential difference is calculated and then divided by two such that an accurate value of the underwater electric potential (first underwater electric potential) can be easily acquired.

### (Item 5)

The offset correction method for the potential difference measuring apparatus according to any one of items 1 to 4, wherein the measuring of the second potential difference includes measuring the second potential difference at a same location at which the first potential difference was measured, with an arrangement of the pair of electrode portions changed from the first position to the second position.

In order to accurately correct the offset of the potential difference caused by the factor other than the test object, it is preferable to measure the first potential difference and the second potential difference in a state in which the values (absolute values) of the underwater electric potentials (first underwater electric potentials) included in the first potential difference and the second potential difference are equal and the positive and negative signs are reversed. Therefore, as described above, the first potential difference and the second potential difference are measured at the same location such that the values (absolute values) of the underwater electric potentials included in the first potential difference and the second potential difference can be equal, and the positive and negative signs can be reversed. Consequently, the offset of the potential difference caused by the surrounding environment of the pair of electrode portions can be accurately corrected. The same location at which the first potential difference was measured includes not only a location at which the first electrode (first measuring electrode) and the second electrode (second measuring electrode) are completely reversed between the first and second positions, but also a location allowing some positional deviation.

### (Item 6)

The offset correction method for the potential difference measuring apparatus according to any one of items 1 to 5, wherein the measuring of the second potential difference includes rotating the pair of electrode portions by 180 degrees from a state in which the pair of electrode portions are placed at the first position to place the pair of electrode portions at the second position, and measuring the second potential difference.

When the pair of electrode portions are placed at the second position, the pair of electrode portions are rotated by 180 degrees, and thus the second potential difference can be easily measured at the second position regardless of the position at which the first position is set. Consequently, it is possible to improve the degree of freedom in the arrangement of the pair of electrode portions at the start of measurement in the potential difference measuring apparatus, and thus convenience for an operator can be improved.

### (Item 7)

The offset correction method for the potential difference measuring apparatus according to item 1, wherein the calculating of the underwater electric potential includes calculating the underwater electric potential at each of a plurality of predetermined angles that are different from each other, in which the offset of the potential difference caused by the factor other than the test object has been corrected, based on the first potential difference measured at each of the plurality of predetermined angles and the second potential difference measured by reversing positions of the pair of electrode portions at each of the plurality of predetermined angles.

The potential difference is acquired while the pair of electrode portions are rotated such that it is possible to acquire the underwater electric potential (first underwater electric potential) at each of the plurality of predetermined angles without providing a plurality of pairs of electrode portions. Consequently, it is possible to reduce or prevent the complexity of the configuration of the apparatus and an increase in the number of components.

### (Item 8)

A potential difference measuring apparatus comprising:
an underwater electric field sensor including a pair of electrode portions and a potential difference measuring unit, the pair of electrode portions including a first electrode and a second electrode configured to measure a potential in water, the potential difference measuring unit being configured to measure a potential difference between the pair of electrode portions; and
a controller configured or programmed to calculate an underwater electric potential in which at least an offset of a potential difference caused by a factor other than a test object has been corrected, based on the potential difference measured by the underwater electric field sensor; wherein
the controller is configured or programmed to:
   place the pair of electrode portions at a predetermined first position and measure a first potential difference between the pair of electrode portions;
   place the pair of electrode portions at a second position at which positions of the first electrode and the second electrode at the first position are reversed, and measure a second potential difference between the pair of electrode portions; and
   calculate the underwater electric potential in which an offset of a potential difference caused by a factor other than a test object has been corrected, based on a measured first potential difference and a measured second potential difference.

Similarly to the offset correction method for the potential difference measuring apparatus described above, it is possible to provide the potential difference measuring apparatus capable of accurately acquiring the underwater electric potential (first underwater electric potential).

### (Item 9)

The potential difference measuring apparatus according to item 8, further comprising:
a rotation mechanism configured to rotate the pair of electrode portions; wherein
the controller is configured or programmed to place the pair of electrode portions at the second position, at which the positions of the first electrode and the second electrode at the first position are reversed, using the rotation mechanism, and measure the second potential difference between the pair of electrode portions.

The rotation mechanism that rotates the pair of electrode portions is provided, and thus it is possible to easily place the pair of electrode portions at the first position and the second position. Consequently, it is possible to provide the potential difference measuring apparatus capable of accurately and easily acquiring the underwater electric potential (first underwater electric potential).

### (Item 10)

The potential difference measuring apparatus according to item 9, wherein the controller is configured or programmed to calculate the underwater electric potential in which the offset of the potential difference caused by the factor other than the test object has been corrected by calculating a difference between a value of the first potential difference and a value of the second potential difference.

Similarly to the offset correction method for the potential difference measuring apparatus described above, it is possible to provide the potential difference measuring apparatus capable of easily and accurately acquiring the underwater electric potential (first underwater electric potential).

### (Item 11)

The potential difference measuring apparatus according to item 9 or 10, wherein the controller is configured or programmed to rotate the pair of electrode portions by 180 degrees using the rotation mechanism after measuring the first potential difference to place the pair of electrode portions at the second position, and measure the second potential difference.

Similarly to the offset correction method for the potential difference measuring apparatus described above, it is possible to provide the potential difference measuring apparatus capable of accurately correcting the offset of the potential difference caused by the factor other than the test object.

### (Item 12)

The potential difference measuring apparatus according to any one of items 8 to 11, wherein the controller is configured or programmed to calculate the underwater electric potential at each of a plurality of predetermined angles that are different from each other, in which the offset of the potential difference caused by the factor other than the test object has been corrected, based on the first potential difference measured at each of the plurality of predetermined angles and the second potential difference measured by reversing positions of the pair of electrode portions at each of the plurality of predetermined angles.

Similarly to the offset correction method for the potential difference measuring apparatus described above, it is possible to acquire the underwater electric potential (first underwater electric potential) at each of the plurality of predetermined angles without providing a plurality of pairs of electrode portions. Consequently, it is possible to provide the potential difference measuring apparatus capable of reducing or preventing the complexity of the configuration of the apparatus and an increase in the number of components.

## Claims

1. An offset correction method for a potential difference measuring apparatus (100), the potential difference measuring apparatus (100) including a pair of electrode portions (10) and a potential difference measuring unit (13), the pair of electrode portions (10) including a first electrode (11a) and a second electrode (12a) configured to measure a potential in water, the potential difference measuring unit (13) being configured to measure a potential difference between the pair of electrode portions (10), the offset correction method comprising:
placing the pair of electrode portions (10) at a predetermined first position and measuring a first potential difference (V1) between the pair of electrode portions (10);
placing the pair of electrode portions (10) at a second position at which positions of the first electrode (11a) and the second electrode (12a) at the first position are reversed, and measuring a second potential difference (V2) between the pair of electrode portions (10); and
calculating an underwater electric potential (Vs1) in which at least an offset of a potential difference caused by a factor other than a test object (80) has been corrected, based on the first potential difference (V1) and the second potential difference (V2).

2. The offset correction method for the potential difference measuring apparatus (100) according to claim 1, wherein the calculating of the underwater electric potential (Vs1) includes calculating the underwater electric potential (Vs1) in which the offset of the potential difference caused by the factor other than the test object (80) has been corrected by calculating a difference between a value of the first potential difference (V1) and a value of the second potential difference (V2).

3. The offset correction method for the potential difference measuring apparatus (100) according to claim 2, wherein the calculating of the underwater electric potential (Vs1) includes calculating the underwater electric potential (Vs1) in which an offset of a potential difference caused by individual differences between the first electrode (11a) and the second electrode (12a), and an offset of a potential difference caused by a surrounding environment of the pair of electrode portions (10) have been corrected by calculating the difference between the value of the first potential difference (V1) and the value of the second potential difference (V2).

4. The offset correction method for the potential difference measuring apparatus (100) according to claim 3, wherein the calculating of the underwater electric potential (Vs1) includes calculating the underwater electric potential (Vs1) by calculating the difference between the value of the first potential difference (V1) and the value of the second potential difference (V2) and then dividing the difference by two.

5. The offset correction method for the potential difference measuring apparatus (100) according to claim 1, wherein the measuring of the second potential difference (V2) includes measuring the second potential difference (V2) at a same location at which the first potential difference (V1) was measured, with an arrangement of the pair of electrode portions (10) changed from the first position to the second position.

6. The offset correction method for the potential difference measuring apparatus (100) according to claim 1, wherein the measuring of the second potential difference (V2) includes rotating the pair of electrode portions (10) by 180 degrees from a state in which the pair of electrode portions (10) are placed at the first position to place the pair of electrode portions (10) at the second position, and measuring the second potential difference (V2).

7. The offset correction method for the potential difference measuring apparatus (100) according to claim 1, wherein the calculating of the underwater electric potential (Vs1) includes calculating the underwater electric potential (Vs1) at each of a plurality of predetermined angles that are different from each other,
in which the offset of the potential difference caused by the factor other than the test object (80) has been corrected, based on the first potential difference (V1) measured at each of the plurality of predetermined angles and the second potential difference (V2) measured by reversing positions of the pair of electrode portions (10) at each of the plurality of predetermined angles.

8. A potential difference measuring apparatus (100) comprising:
an underwater electric field sensor (1) including a pair of electrode portions (10) and a potential difference measuring unit (13), the pair of electrode portions (10) including a first electrode (11a) and a second electrode (12a) configured to measure a potential in water, the potential difference measuring unit (13) being configured to measure a potential difference between the pair of electrode portions (10); and
a controller (3) configured or programmed to calculate an underwater electric potential (Vs1) in which at least an offset of a potential difference caused by a factor other than a test object (80) has been corrected, based on the potential difference measured by the underwater electric field sensor (1); wherein
the controller (3) is configured or programmed to:
place the pair of electrode portions (10) at a predetermined first position and measure a first potential difference (V1) between the pair of electrode portions (10);
place the pair of electrode portions (10) at a second position at which positions of the first electrode (11a) and the second electrode (12a) at the first position are reversed, and measure a second potential difference (V2) between the pair of electrode portions (10); and
calculate the underwater electric potential (Vs1) in which an offset of a potential difference caused by a factor other than a test object (80) has been corrected, based on a measured first potential difference (V1) and a measured second potential difference (V2).

9. The potential difference measuring apparatus (100) according to claim 8, further comprising:
a rotation mechanism (2) configured to rotate the pair of electrode portions (10); wherein
the controller (3) is configured or programmed to place the pair of electrode portions (10) at the second position, at which the positions of the first electrode (11a) and the second electrode (12a) at the first position are reversed, using the rotation mechanism (2) and measure the second potential difference (V2) between the pair of electrode portions (10).

10. The potential difference measuring apparatus (100) according to claim 9, wherein the controller (3) is configured or programmed to calculate the underwater electric potential (Vs1) in which the offset of the potential difference caused by the factor other than the test object (80) has been corrected by calculating a difference between a value of the first potential difference (V1) and a value of the second potential difference (V2).

11. The potential difference measuring apparatus (100) according to claim 10, wherein the controller (3) is configured or programmed to rotate the pair of electrode portions (10) by 180 degrees using the rotation mechanism (2) after measuring the first potential difference (V1) to place the pair of electrode portions (10) at the second position, and measure the second potential difference (V2).

12. The potential difference measuring apparatus (100) according to claim 8, wherein the controller (3) is configured or programmed to calculate the underwater electric potential (Vs1) at each of a plurality of predetermined angles that are different from each other,
in which the offset of the potential difference caused by the factor other than the test object (80) has been corrected, based on the first potential difference (V1) measured at each of the plurality of predetermined angles and the second potential difference (V2) measured by reversing positions of the pair of electrode portions (10) at each of the plurality of predetermined angles.
